# EUROPÄISCHE PATENTANMELDUNG

(11) **EP 2 206 489 A1**
(43) Veröffentlichungstag der Anmeldung: **14.07.2010**
(21) Anmeldenummer: 09000134.8
(22) Anmeldetag: 08.01.2009
(51) Int. Cl.: A61K 8/02, A61K 8/34, A61K 8/37, A61K 8/39, A61K 8/60, A61K 8/92, A61Q 19/00

(54) **Fließfähige Emulsionskonzentrate**

(71) Anmelder: Cognis IP Management GmbH, 49589 Düsseldorf (DE)
(72) Erfinder: Schulte, Petra, 50733 Köln (DE); Strauss, Gabriele, 40789 Düsseldorf (DE); Kawa, Rolf, 40789 Monheim (DE); Stork, Anja, 50733 Köln (DE)

(57) **Zusammenfassung**

Die vorliegende Erfindung betrifft bei niedrigen Temperaturen fließfähige und pumpbare Emulsionskonzentrate mit guten sensorischen Eigenschaften und guter Lagerstabilität, die insbesondere für kosmetische Tücher geeignet sind, und die keine ethoxylierten Substanzen enthalten.

## Beschreibung

### Gebiet der Erfindung

Die vorliegende Erfindung betrifft bei niedrigen Temperaturen fließfähige und pumpbare Emulsionskonzentrate mit guten sensorischen Eigenschaften und guter Lagerstabilität, die insbesondere für kosmetische Tücher geeignet sind, und bei denen auf den Einsatz von ethoxylierten Rohstoffen verzichtet werden soll.

### Stand der Technik

Emulsionskonzentrate des Marktes basieren in der Regel auf ethoxylierten Rohstoffen, was aus ökologischer Sicht kritisch gesehen wird.

Für EO-freie, hydrophile Emulslonskonzentrate ist eine Kombination bekannt, die auf wasserunlöslichen Ölkomponenten, Emulgatoren und Polyolen basiert und z. B. unter der Bezeichnung Emulgade® CPE (Handelsname) erhältlich ist.

Nachteilig an diesen Emulsionskonzentraten sind ihr Viskositätsanstieg bei niedrigen Temperaturen, insbesondere bei Temperaturen von unter 15°C und ihre sensorischen Eigenschaften.

WO 20081019773 A1 beschreibt Emulsionskonzentrate mit wasserunlöslichen Komponenten, hydrophilen nichtionischen Emulgatoren, lipophilen Coemulgatoren, Polyolen und Wasser. Dabei werden Emulgatoren auf Basis ethoxylierter Substanzen verwendet. Weiterhin wird eine Fließ- und Pumpfähigkeit der Emulsionskonzentrate bei Raumtemperatur angegeben.

Aufgabe der vorliegenden Erfindung war es, Emulsionskonzentrate ohne ethoxylierte Rohstoffe zur Verfügung zu stellen, die bei niedrigen Temperaturen unterhalb 15°C fließfähig und pumpbar sind und welche gute sensorische Eigenschaften aufweisen.
Als fließfähig und pumpbar werden erfindungsgemäß Emulsionskonzentrate bezeichnet, deren Viskosität bei 15°C unterhalb 30000 mPas, gemessen mit einem Brookfield Rotationsviskosimeter (Typ RVF, Spindel 5,10 UpM), liegt.

Diese Emulsionskonzentrate können unverdünnt auf Substrate aufgebracht werden, werden aber normalerweise verdünnt. Im einfachsten Fall erfolgt die Verdünnung mit Wasser. Es ist eine weitere Aufgabe der Erfindung, Emulsionskonzentrate bereit zu stellen, die nach Verdünnung lagerstabil sind, was im Allgemeinen gewährleistet ist, wenn die Teilchengröße, gemessen mit einem Coulter LS, kleiner 100 nm ist und die Verdünnungen transparent sind und nicht separieren.

### Beschreibung der Erfindung

Überraschenderweise wurde gefunden, dass Emulsionskonzentrate der vorliegenden Erfindung diese Probleme lösen.

Gegenstand der vorliegenden Erfindung ist ein Emulsionskonzentrat, enthaltend folgende Komponenten:
(A) Alkyl- und/oder Alkenylollgoglykosid(e) mit 8 bis 22 Kohlenstoffatomen im Alk(en)ylrest,
(B) Polyglycerolester,
(C) Polyol(e),
(D) Glycerid(e),
(E) wasserunlösliche Komponente(n) mit einer Polarität von etwa 30 - 40 mN/m und
(F) wasserunlösliche Komponente(n) mit einer Polarität von etwa 15 - 25 mN/m,
wobei
die Komponenten (A), (B) und (C) ein Gewichtsverältnis A: B : C von 1 : (0,9 -1,1) : (0,9 - 1,1) aufweisen,
die Komponenten (A) und (D) ein Gewichtsverhältnis A : D von 6 : 1 bis 3 :1 aufweisen,
die Komponenten (E) und (F) ein Gewichtsverhältnis E : F von 1 : 1 bis 1 : 3 aufweisen, und der Wasseranteil des Emulsionskonzentrats 10 bis 20 Gew.-% beträgt.

Werden diese Bedingungen eingehalten, können Emulsionskonzentrate erhalten werden, welche bei 15°C eine Viskosität von unterhalb 30000 mPa*s aufweisen, bestimmt mittels eines Brookfield Rotationsviskosimeters (Typ RVF, Spindel 5, 10 UpM). Bevorzugt weisen die erfindungsgemäßen Emulsionskonzentrate bei 15°C eine Viskosität von unterhalb 20000 mPa*s, weiter bevorzugt unter 15000 mPa*s, auf.

Sowohl die erfindungsgemäßen Emulsionskonzentrate als auch deren Verdünnungen weisen eine gute Lagerstabilität auf, welche sich darin äußert, dass bspw. nach 5 %-iger Verdünnung mit Wasser nach einer Woche die Verdünnungen transparent sind und die mittlere Teilchengröße, bestimmt mittels eines Coulter LS, kleiner 100 nm ist.

Die vorliegende Erfindung betrifft auch kosmetische und/oder pharmazeutische Zubereitungen, welche durch Verdünnen des erfindungsgemäßen Emulsionskonzentrats erhalten werden.

Weiterhin betrifft die vorliegende Erfindung Papier-, Vlies- oder Gewebe-Produkte zur Körperpflege und/oder -reinigung, wie z. B. kosmetische Tücher, welche das Emulsionskonzentrat oder die kosmetische und/oder pharmazeutische Zubereitung enthalten.

Überraschenderweise wurde gefunden, dass Emulsionskonzentrate hergestellt werden können, die bei niedrigen Temperaturen fließfähig und pumpbar sind, gute sensorische Eigenschaften aufweisen und lagerstabil sind, ohne dass bei der Formulierung ethoxylierte Substanzen verwendet werden müssen. Diese Wirkungen können durch das Zusammenspiel der Komponenten und deren Mengenverhältnisse, wie in Anspruch 1 angegeben, überraschenderweise in dem Emulsionskonzentrat erzielt werden.

### Wasserunlösliche Komponenten (Komponenten (E) und (F))

Ein wesentliches Merkmal der vorliegenden Erfindung ist die Zusammenstellung der lipophilen Phase hinsichtlich der Komponenten (E) und (F). Es ist erfindungsgemäß erforderlich, dass ein Teil der lipophilen Phase durch Komponenten mit niedriger Polarität gebildet wird (Komponente (E)) und ein Teil durch Komponenten mit hoher Polarität (Komponente (F)).

Dementsprechend weist erfindungsgemäß ein Teil der lipophilen Phase eine mittlere Polarität von 15 - 25 mN/m und ein anderer Teil eine mittlere Polarität 30 - 40 mN/m auf.
Insoweit die Wirkungen der vorliegenden Erfindung nicht beeinträchtigt werden und die Bedingungen bezüglich (E) und (F) eingehalten werden, können zusätzlich Ölkomponenten mit anderer Polarität enthalten sein.

Die Polarität der wasserunlöslichen Ölkomponenten (E) und (F) wird über die Grenzflächenspannung angegeben. Die Grenzflächenspannung ist die diejenige Kraft, die an einer gedachten, in der Grenzfläche zwischen zwei Phasen befindlichen Linie der Länge von einem Meter wirkt. Die physikalische Einheit für die Grenzflächenspannung errechnet sich klassisch nach der Beziehung Kraft/Länge und wird gewöhnlich In mN/m (Millinewton geteilt durch Meter) wiedergegeben. Sie hat ein positives Vorzeichen, wenn sie das Bestreben hat, die Grenzfläche zu verkleinern, im umgekehrten Fall hat sie ein negatives Vorzeichen. Kleinere positive Werte bedeuten eine höhere Polarität. Die Grenzflächenspannung wird erfindungsgemäß nach der ASTM Methode D971-99a (reapproved 2004) bestimmt.

Es war nicht zu erwarten, dass durch die erfindungsgemäße Polaritätenverteilung in der lipophilen Phase die Fließfähigkeit der Emulsionskonzentrate bei niedrigen Temperaturen verbessert werden konnte und gleichzeitig andere Vorteile erzielt werden konnten, wie gute sensorische Eigenschaften, Verzicht auf ethoxylierte Rohstoffe und Lagerstabilität, wenn auch die anderen Merkmale der Erfindung eingehalten werden.

Der Gehalt der Komponente (E), bezogen auf das Emulsionskonzentrat, beträgt in der Regel etwa 3 bis etwa 25 Gew.-%, bevorzugt 5 - 20 Gew.- %., noch bevorzugter 7 - 15 Gew.-%.

Der Gehalt der Komponente (F), bezogen auf das Emulsionskonzentrat, beträgt in der Regel etwa 15 bis etwa 40 Gew.-%, bevorzugt 20 - 35 Gew.- %.
Das Gewichtsverhältnis der Komponenten (E) und (F) zueinander beträgt erfindungsgemäß etwa 1:1 bis etwa 1 : etwa 3.

Die wasserunlöslichen Ölkomponenten (E) und (F) können Öle, Fette, Wachse sowie beliebige Mischungen daraus enthalten.

Als wasserunlösliche Ölkomponenten (E) und (F) eignen sich alle bei 30°C flüssigen Fettstoffe oder Fettstoffgemische, d. h. auch Gemische aus flüssigen und darin gelösten, festen Fettstoffen oder Paraffinen, solange diese Gemische bei 30°C flüssig sind oder deren Viskosität (20°C) unter 20 Pas liegt (gemessen mit einem Brookfield Rotationsviskosimeter Typ RVF, Spindel 4, 10 UpM), wie z. B. flüssige Kohlenwasserstoffe, Dialkylether, Fettsäureester mit 12 - 44 C-Atomen, Dialkylcarbonate, Guerbetalkohole und Siliconöle oder deren Mischungen.

Nachfolgend werden Beispiele für Komponenten der lipophilen Phase angegeben. Der Fachmann kann hieraus die für die Erfindung geeigneten Komponenten anhand der Polarität der Komponenten auswählen, indem er die Polarität der Substanzen in Handbüchem nachschlägt oder wie oben beschrieben nach der ASTM Methode D971-99a (reapproved 2004) bestimmt. Die nachfolgende Aufzählung umfasst auch Substanzen, die nicht in die genannten Pofaritätsbereiche der Komponenten (E) und (F) fallen. Diese Substanzen können aber, wie oben erwähnt, zusätzlich zu den Komponenten (E) und (F) enthalten sein, solange sie die Wirkung der Erfindung nicht beeinträchtigen.

Unter dem Begriff "Öle" (synonym verwendet: Ölkomponente) werden wasserunlösliche, bei 30°C flüssige, organische Verbindungen mit relativ niedrigem Dampfdruck bezeichnet. Das gemeinsame Merkmal der Öle ist nicht ihre übereinstimmende chemische Konstitution, sondere ihre ähnliche physikalische Konsistenz.

Als Ölkomponenten sind beispielsweise die nachstehend genannten Verbindungsklassen geeignet, soweit diese bei 30°C flüssig sind. So z.B. Guerbetalkohole auf Basis von Fettalkoholen mit 6 bis 18, vorzugsweise 8 bis 10 Kohlenstoffatomen (z. B. Eutanol^{®} G), Ester von linearen C₆-C₂₂-Fettsäuren mit linearen oder verzweigten C₆-C₂₂-Fettalkoholen (z.B. Cetiol^{®} Sensoft) bzw. Ester von verzweigten C₆-C₁₃-Carbonsäuren mit linearen oder verzweigten C₆-C₂₂-Feffalkoholen, wie z.B. Myristylmyristat, Myristylpalmitat, Myristylstearat, Myristylisostearat, Myristyloleat, Myristylbehenat, Myristylerucat, Cetylmyristat, Cetylpalmitat, Cetylstearat, Cetylisostearat, Cetyloleat, Cetylbehenat, Cetylerucat, Stearylmyristat, Stearylpalmitat, Stearylstearat, Stearylisostearat, Stearyloleat, Stearylbehenat, Stearylerucat, Isostearylmyristat, Isostearylpalmitat, Isostearylstearat, Isostearylisostearat, Isostearyloleat, Isostearylbehenat, Isostearyloleat, Oleylmyristat, Oleylpalmitat, Oleylstearat, 0leylisostearat, Oloyloleat, Oleylbehenat, Oleylerucat, Behenylmyristat, Behenylpalmitat, Behenylstearat, Behenylisostearat, Behenyloleat, Behenylbehenat, Behenylerucat, Erucylmyristat, Erucylpalmitat, Erucylstearat, Erucylisostearat, Erucyloleat, Erucylbehenat, Erucylerucat und Hexyldecylstearat (Eutanol® G 16 S). Daneben eignen sich Ester von linearen C₆-C₂₂-Fettsäuren mit verzweigten Alkoholen, insbesondere 2- Ethylhexanol, Ester von C₃-C₃₈-Alkylhydroxycarbonsäuren mit linearen oder verzweigten C₆-C₂₂-Feftalkoholen - insbesondere Dioctylmalat -, Ester von linearen und/oder verzweigten Fettsäuren mit mehrwertigen Alkoholen (wie z.B. Propylenglycol, Dimerdiol oder Trimertriol) und/oder Guerbetalkoholen, Triglyceride auf Basis C₆-C₁₀-Fettsäuren, flüssige Mono-/Di-/Trigiyceridmischungen auf Basis von C₆-C₁₈-Fettsäuren, Ester von C₆-C₂₂-Fettalkoholen und/oder Guerbetalkoholen mit aromatischen Carbonsäuren, insbesondere Benzoesäure, Ester von C₂-C₁₂-Dicarbonsäuren mit linearen oder verzweigten Alkoholen mit 1 bis 22 Kohlenstoffatomen oder Polyolen mit 2 bis 10 Kohlenstoffatomen und 2 bis 6 Hydroxylgruppen, pflanzliche Öle, verzweigte primäre Alkohole, substituierte Cyclohexane wie z.B. 1 ,3-Dialkylcyclohexane, lineare und verzweigte C₆-C₂₂-Fettalkoholcarbonate, wie z.B. Dicaprylyl Carbonate (Cetiol® CC), Guerbetcarbonate auf Basis von Fettalkoholen mit 6 bis 18, vorzugsweise 8 bis 10 C Atomen, Ester der Benzoesäure mit linearen und/oder verzweigten C₆-C₂₂-Alkoholen (z.B. Finsolv® TN), lineare oder verzweigte, symmetrische oder unsymmetrische Dialkylether mit 6 bis 22 Kohlenstoffatomen pro Alkylgruppe, wie z.B. Dicaprylyl Ether (Cetiol® OE), Ringöffnungsprodukte von epoxidierten Fettsäureestem mit Polyolen (Hydagen® HSP, Sovermol® 750, Sovermol® 1102), Siliconöle (Cyclomethicone, Siliciummethicontypen u.a. und/oder aliphatische bzw. naphthenische Kohlenwasserstoffe, wie z.B. wie Mineralöl, Vaseline, Petrolatum, Squalan, Squalen oder Dialkylcyclohexane.

Als weitere Ölkörper kommen beispielsweise Silikonöle in Frage. Sie können als cyclische und/oder lineare Silikonöle vorliegen. Silikonöle sind hochmolekulare synthetische polymere Verbindungen, in denen Silicium- Atome über Sauerstoff-Atome ketten-und/oder netzartig verknüpft und die restlichen Valenzen des Siliciums durch Kohlenwasserstoff-Reste (meist Methyl-, seltener Ethyl-, Propyl-, PhenylGruppen u. a.) abgesättigt sind. Systematisch werden die Silikonöle als Polyorganosiloxane bezeichnet. Die methylsubstituierien Polyorganosiloxane, welche die mengenmäßig bedeutendsten Verbindungen dieser Gruppe darstellen und sich durch die folgende Strukturformel 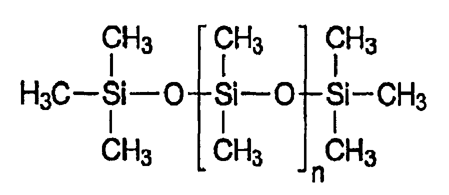 auszeichnen werden auch als Polydimethylsiloxan bzw. Dimethicon (INCI) bezeichnet. Dimethicone gibt es in verschiedenen Kettenlängen bzw. mit verschiedenen Molekulargewichten.

Vorteilhafte Polyorganosiloxane im Sinne der vorliegenden Erfindung sind beispielsweise Dimethylpolysiloxan [Poly (dimethylsiloxan)], welche beispielsweise unter den Handels- bezeichnungen Abil 10 bis 10 000 bei Evonik Goldschmidt erhältlich sind. Ferner vorteilhaft sind Phenylmethylpolysiloxan (INCI: Phenyl Dimethicon, Phenyl Trimethicon), cyclische Silikone (Octamethylcyclotetrasiloxan bzw. Decamethylcyclopentasiloxan), welche nach INCI auch als Cyclomethicon bezeichnet werden, aminomodifizierte Silikone (INCI: Amodimethicone) und Silikonwachse, z. B. Polysiloxan-Polyalkylen-Copolymere (INCI: Stearyl Dimethicon und Cetyl Dimethicon) und Dialkoxydimethylpolysiloxane (Stearoxy Dimethicon und Behenoxy Stearyl Dimethicon), welche als verschiedene Abil-Wax-Typen bei Evonik Goldschmidt erhältlich sind. Aber auch andere Silikonöle sind vorteilhaft im Sinne der vorliegenden Erfindung zu verwenden, beispielsweise Cetyidimethicon, Hexamethylcyclo-trisiloxan, Polydimethylsiloxan, Poly (methylphenylsiloxan). Erfindungsgemäß besonders bevorzugte Silikone sind Dimethicon und Cyclomethicon.

Als Ölkomponenten eignen sich auch Polycarbonate, wie beispielsweise in WO 03/041676 beschrieben, auf die hier ausdrücklich Bezug genommen wird.

Als Polycarbonate besonders geeignet ist das unter der INCI Bezeichnung Hydrogenated Dimer Dillnoleyl / Dlmethylcarbonate Copolymer, welches als Handelsprodukt Cosmedia^{®} DC von Cognis GmbH erhältlich ist.

Die Dialkylcarbonate und Dialkylether können symmetrisch oder unsymmetrisch, verzweigt oder unverzweigt, gesättigt oder ungesättigt sein und lassen sich nach Reaktionen, die aus dem Stand der Technik hinlänglich bekannt sind, herstellen.

Erfindungsgemäß einsetzbar sind u. a. auch Kohlenwasserstoffe, vorzugsweise mit einer Kettenlänge 8 bis 40 C-Atomen. Sie können verzweigt oder unverzweigt sein, gesättigt oder ungesättigt. Unter diesen sind verzweigte, gesättigte C₈-C₄₀-Alkane bevorzugt. Es können sowohl Reinsubstanzen eingesetzt werden als auch Substanzgemische. Üblicherweise handelt es sich um Substanzgemische verschiedener isomerer Verbindungen. Zusammensetzungen, die Alkane mit 10 bis 30, vorzugsweise 12 bis 20, und besonders bevorzugt 16 bis 20 Kohlenstoffatome aufweisen, sind besonders geeignet, und unter diesen ein Gemisch aus Alkanen, welches wenigstens 10 Gew.-% verzweigte Alkane bezogen auf die Gesamtmenge der Alkane enthält. Vorzugsweise handelt es sich um verzweigte, gesättigte Alkane. Besonders gut geeignet sind Gemische aus Alkanen, welche mehr als 1 Gew.-% 5,8-Diethyldodecan und/oder mehr als 1 Gew.-% Didecen enthalten.

Es kann erfindungsgemäß eine Öl oder ein Gemisch aus mehreren Ölkomponenten verwendet werden.

Unter dem Begriff **Wachs** (synonym verwendet: Wachskomponente) werden üblicherweise alle natürlichen oder künstlich gewonnenen Stoffe und Stoffgemische mit folgenden Eigenschaften verstanden: sie sind von fester bis brüchig harter Konsistenz, grob bis feinteilig, durchscheinend bis trüb und schmelzen oberhalb von 30°C ohne Zersetzung. Sie sind schon wenig oberhalb des Schmelzpunktes niedrigviskos und nicht fadenziehend und zeigen eine stark temperaturabhängige Konsistenz und Löslichkeit. Erfindungsgemäß einsetzbar ist eine Wachskomponente oder ein Gemisch von Wachskomponenten, die bei 30°C oder darüber schmelzen.

Als Wachse können erfindungsgemäß auch Fette und fettähnliche Substanzen mit wachsartiger Konsistenz eingesetzt werden, solange sie den geforderten Schmelzpunkt haben. Hierzu gehören u.a. Fette (Triglyceride), sowie natürliche und synthetische Wachse oder beliebige Gemische dieser Substanzen.

Unter **Fetten** versteht man Triacylglycerine, also die Dreifachester von Fettsäuren mit Glycerin. Bevorzugt enthalten sie gesättigte, unverzweigte und unsubstituierte Fettsäurereste. Hierbei kann es sich auch um Mischester, also um Dreifachester aus Glycerin mit verschiedenen Fettsäuren handeln. Erfindungsgemäß einsetzbar sind so genannte gehärtete Fette und Öle, die durch Partialhydrierung gewonnen werden. Pflanzliche gehärtete Fette und Öle sind bevorzugt, z. B. gehärtetes Rizinusöl, Erdnussöl, Sojaöl, Rapsöl, Rübsamenöl, Baumwollsaatöl, Sojaöl, Sonnenblumenöl, Palmöl, Palmkernöl, Leinöl, Mandelöl, Maisöl, Olivenöl, Sesamöl, Kakaobutter und Kokosfett.

Geeignet sind u.a. die Dreifachester von Glycerin mit C₁₂-C₆₀-Fettsäuren und insbesondere C₁₂-C₃₅-Fettsäuren. Hierzu zählt gehärtetes Rizinusöl, ein Dreifachester aus Glycerin und einer Hydroxystearinsäure, der beispielsweise unter der Bezeichnung Cutina® HR im Handel Ist. Ebenso geeignet sind Glycerintristearat, Glycerintribehenat (z. B. Syncrowax® HRC), Glycerintripalmitat oder die unter der Bezeichnung Syncrowax® WGLC bekannten Triglycerid-Gemische, mit der Vorgabe, dass der Schmelzpunkt der Wachskomponente bzw. des Gemisches bei 30 °C oder darüber liegt.

Als Wachskomponenten sind erfindungsgemäß insbesondere Mono- und Diglyceride bzw. Mischungen dieser Partialglyceride einsetzbar. Zu den erfindungsgemäß einsetzbaren Glycefidgemischen zählen die von der Cognis GmbH vermarkteten Produkte Novata AB und Novata B (Gemisch aus C₁₂-C₁₈-Mono-, Di- und Triglyceriden) sowie Cutina MD oder Cutina GMS (Glycerylstearat).

Weitere Wachskomponenten sind aus der Gruppe der C6-C22-Fettsäureester des Pentaerythrits, des Dipentaerythrits, des Tripentaerythrifs oder eines beliebigen Gemisches dieser Ester, die einen Schmelzpunkt von wenigstens 30°C aufweisen. Ein bevorzugtes Pentaerythritsestergemisch besteht aus einem Anteil von 5 - 35 Gew.-% Monoester, 20 - 50 Gew.-% Diester und 25 - 50 Gew.-% Triester, und ggf. Tetraester. Besonders bevorzugt ist ein Gehalt an 10 - 25 Gew.-% Monoester, 25 - 40 Gew.-% Diester und 30 - 45 Gew.-% Triester, und ggf. Tetraester und ganz besonders bevorzugt 12 - 19 Gew.-% Monoester, 25 - 35 Gew.-% Diester und 30 - 40 Gel.-% Triester und 6 - 11 Gew.-% Tetraester. Zu dem erfindungsgemäß einsetzbarem Pentaerythritsestergemisch zählt das von der Cognis GmbH vermarktete Produkt ist Cutina^{®} PES.

Zu den erfindungsgemäß als Wachskomponente einsetzbaren Fettalkoholen zählen die C₁₂-C₅₀-Fettalkohole. Die Fettalkohole können aus natürlichen Fetten, Ölen und Wachsen gewonnen werden, wie beispielsweise Myristylalkohol, 1-Pentadecanol, Cetylalkohol, 1-Heptadecanol, Stearylalkohol, 1-Nonadecanol, Arachidylalkohol, 1-Heneicosanol, Behenylalkohol, Brassidylalkohol, Lignocerylalkohol, Cerylalkohol oder Myricylalkohol. Erfindungsgemäß bevorzugt sind gesättigte unverzweigte Fettalkohole. Aber auch ungesättigte, verzweigte oder unverzweigte Fettalkohole können erfindungsgemäß als Wachskomponente verwendet werden, solange sie den geforderten Schmelzpunkt aufweisen. Erfindungsgemäß einsetzbar sind auch Feftalkoholschnitte, wie sie bei der Reduktion natürlich vorkommender Fette und Öle wie z. B. Rindertalg, Erdnußöl, Rüböl, Baumwollsaatöl, Sojaöl, Sonnenblumenöl, Palmkernöl, Leinöl, Rizinusöl, Maisöl, Rapsöl, Sesamöl, Kakaobutter und Kokosfett anfallen. Es können aber auch synthetische Alkohole, z. B. die linearen, geradzahligen Fettalkohole der Ziegler-Synthese (Alfole) oder die teilweise verzweigten Alkohole aus der Oxosynthese (Dobanole) verwendet werden. Erfindungsgemäß besonders bevorzugt geeignet sind C₁₄-C₂₂-Feftalkohole, die beispielsweise von der Cognis GmbH unter der Bezeichnung Lanette 18 (C₁₈-Alkohol), Lanette 16 (C₁₆-Alkohol), Lanette 14 (C₁₄-Alkohol), Lanette O (C₁₆/C₁₈-Alkohol) und Lanette 22 (C₁₈/C₂₂-Alkohol) vermarktet werden. Fettalkohole verleihen den Zubereitungen ein trockeneres Hautgefühl als Triglyceride und sind daher gegenüber letzteren bevorzugt.

Als Wachskomponenten können auch C₁₄-C₄₀-Fettsäuren oder deren Gemische eingesetzt werden. Hierzu gehören beispielsweise Myristin-, Pentadecan-, Palmifin-, Margarin-, Stearin-, Nonadecan-, Arachin-, Behen-, Lignocerin-, Cerotin-, Melissin-, Eruca- und Elaeostearinsäure sowie substituierte Fettsäuren, wie z. B. 12-HydroxystearInsäure, und die Amide oder Monoethanolamide der Fettsäuren, wobei diese Aufzählung beispielhaften und keinen beschränkenden Charakter hat.

Erfindungsgemäß verwendbar sind beispielsweise **natürliche pflanzliche Wachse**, wie Candelillawachs, Camaubawachs, Japanwachs, Espartograswachs, Korkwachs, Guarumawachs, Reiskeimölwachs, Zuckerrohrwachs, Ouricurywachs, Montanwachs, Sonnenblumenwachs, Fruchtwachse wie Orangenwachse, Zitronenwachse, Grapefruitwachs, Lorbeerwachs (=Bayberrywax) und **tierische Wachse**, wie z. B. Bienenwachs, Schellackwachs, Walrat, Wollwachs und Bürzelfett. Im Sinne der Erfindung kann es vorteilhaft sein, hydrierte oder gehärtete Wachse einzusetzen. Zu den erfindungsgemäß verwendbaren natürlichen Wachsen zählen auch die **Mineralwachse**, wie z. B. Ceresin und Ozokerit oder die petrochemischen Wachse, wie z. B. Petrolatum, Paraffinwachse und Mikrowachse. Als Wachskomponente sind auch **chemisch modifizierte Wachse**, insbesondere die Hartwachse, wie z. B. Montanesterwachse, Sasolwachse und hydrierte Jojobawachse einsetzbar. Zu den **synthetischen Wachsen**, die erfindungsgemäß einsetzbar sind, zählen beispielsweise wachsartige Polyalkylenwachse und Polyethylenglycolwachse. Pflanzliche Wachse sind erfindungsgemäß bevorzugt.

Die Wachskomponente kann ebenso gewählt werden aus der Gruppe der Wachsester aus gesättigten und/oder ungesättigten, verzweigten und/oder unverzweigten Alkancarbonsäuren und gesättigten und/oder ungesättigten, verzweigten und/oder unverzweigten Alkoholen, aus der Gruppe der Ester aus aromatischen Carbonsäuren, Dicarbonsäuren, Tricarbonsäuren bzw. Hydroxycarbonsäuren (z. B. 12-Hydroxystearinsäure) und gesättigten und/oder ungesättigten, verzweigten und/oder unverzweigten Alkoholen, sowie ferner aus der Gruppe der Lactide langkettiger Hydroxycarbonsäuren. Beispiel solcher Ester sind die C₁₆-C₄₀-Alkylstearate, C₂₀-C₄₀-Alkylstearate (z. B. Kesterwachs K82H), C₂₀-C₄₀-Dialkylester von Dimersäuren, C₁₈-C₃₈-Alkylhydroxystearoylstearate oder C₂₀-C₄₀-Alkylerucate. Ferner sind C₃₀-C₅₀-Alkylbienenwachs, Tristearylcitrat, Triisostearylcitrat, Stearylheptanoat, Stearyloctanoat, Trilaurylcitrat, Ethylenglycoldipalmitat, Ethylenglycoldistearat, Ethylenglykoldi(12-hydroxystearat), Stearylstearat, Palmitylstearat, Stearylbehenat, Cetylester, Cetearylbehenat und Behenylbehenat einsetzbar. Auch Fettsäurepartialglyceride, d. h. technische Mono- und/oder Diester des Glycerins mit Fettsäuren mit 12 bis 18 Kohlenstoffatomen wie etwa Glycerinmono/dilaurat, -palmitat, - myristat oderstearat kommen hierfür in Frage.

Als Wachse eignen sich weiterhin **Perlglanzwachse**. Als Perlglanzwachse, insbesondere für den Einsatz in tensidischen Formulierungen, kommen beispielsweise in Frage: Alkylenglycolester, speziell Ethylenglycoldistearat; Fettsäurealkanolamlde, speziell Kokosfettsäurediethanolamid; Partialglyceride, speziell Stearinsäuremonoglycerid; Ester von mehrwertigen, gegebenenfalls hydroxy-substttulerte Carbonsäuren mit Fettalkoholen mit 6 bis 22 Kohlenstoffatomen, speziell langkettige Ester der Weinsäure; Fettstoffe, wie beispielsweise Fettalkohole, Fettketone, Fettaldehyde, Fettether und Fettcarbonate, die in Summe mindestens 24 Kohlenstoffatome aufweisen, speziell Lauron und Distearylether, Fettsäuren wie Stearinsäure, Hydroxystearinsäure oder Behensäure, Ringöffnungsprodukte von Olefinepoxiden mit 12 bis 22 Kohlenstoffatomen mit Fettalkoholen mit 12 bis 22 Kohlenstoffatomen und/oder Polyolen mit 2 bis 15 Kohlenstoffatomen und 2 bis 10 Hydroxylgruppen sowie deren Mischungen.

Es kann erfindungsgemäß ein Wachs oder ein Gemisch aus mehreren Wachsen verwendet werden.

### Komponente (A) - Emulgator

Als Emulgator (Komponente (A)) werden erfindungsgemäß Alkyl- und/oder Alkenyl(oligo)glykosid(e) mit 8 bis 22 Kohlenstoffatomen im Alk(en)ylrest eingesetzt.

C₈-C₂₂-Alkylmono- und -oligoglycoside, ihre Herstellung und ihre Verwendung sind aus dem Stand der Technik bekannt. Ihre Herstellung erfolgt insbesondere durch Umsetzung von Glucose oder Oligosacchariden mit primären Alkoholen mit 8 bis 22 C-Atomen. Bezüglich des Glycosidrestes gilt, dass sowohl Monoglycoside, bei denen ein zyklischer Zuckerrest glycosidisch an den Fettalkohol gebunden ist, als auch oligomere Glycoside mit einem Oligomerisationsgrad bis vorzugsweise etwa 8 geeignet sind. Der Oligomerisierungsgrad ist dabei ein statistischer Mittelwert, dem eine für solche technischen Produkte übliche Homologenverteilung zugrunde liegt. Produkte, die unter der Bezeichnung Plantacare^{®} zur Verfügung stehen, enthalten eine glucosidisch gebundene C₈-C₁₆-Alkylgruppe an einem Ollgoglucosidrest, dessen mittlerer Oligomerisationsgrad bei 1 bis 2 liegt. Ein bevorzugtes Beispiel ist Laurylglucosid. Ebenfalls erfindungsgemäß bevorzugt ist Komponente (A) ein Alkylollgoglykosidgemisch mit einem Verhältnis von C12-Alkylrest zu C14-Alkylrest von etwa 7: etwa 3.

Die Emulsionskonzentrate können die Komponente (A) in Mengen von 1 bis 20, vorzugsweise 10 bis 20, insbesondere 12 bis 18 Gew.% - bezogen auf das Gesamtgewicht des Konzentrats - enthalten.

### Komponente (B) - Lipophiler Coemulgator

Komponente (B) ist erfindungsgemäß ein Polyglycerol- bzw. Polyglycerinester als lipophiler Coemulgator.

Die lipophilen Coemulgatoren (B) sind nichtionogene, polare Lipidstoffe mit einer oder mehreren Hydroxylgruppen, die in Wasser unlöslich oder nur dispergierbar sind und die aufgrund ihrer niedrigen Hydrophilie allein nicht zur Herstellung von Öl-in-Wasser-Emulsionen geeignet sind.

Lipophile Coemulgatoren weisen vorzugsweise einen HLB-Wert von < 10 auf.

Als Polyglycerinester können beispielsweise die folgenden Verbindungen eingesetzt werden.

Typische Beispiele für geeignete Polyglycerinester sind Polyglyceryl-4 Diisostearate/ Polyhydroxystearate/Sebacate (Isolan GPS), Polyglyceryl-2 Dipolyhydroxystearate (Dehymuls PGPH), Polyglycerin-3-Diisostearate (Lameform TGI), Polyglyceryl-4 Isostearate (Isolan GI 34), Polyglyceryl-3 Oleate, Diisostearoyl Polyglyceryl-3 Diisostearate (Isolan PDI), Polyglyceryl-3 Methylglucose Distearate (Tego Care 450), Polyglyceryl-3 Beeswax (Cera Bellina), Polyglyceryl-4 Caprate (Polyglycerol Caprate T2010190), Polyglyceryl-3 Cetyl Ether (Chimexane NL), Polyglyceryl-3 Distearate (Cremophor GS 32) und Polyglyceryl Polyricinoleate (Admul WOL 1403) Polyglyceryl Dimerate Isostearate sowie deren Gemische. Bevorzugt sind erfindungsgemäß Polyglycerinester von Polyhydroxystearinsäure. Polyglyceryl-2 Dlpolyhydroxystearat ist ein erfindungsgemäß bevorzugtes Beispiel.

Die Emulsionskonzentrate können die Komponente (B) in Mengen von 1 bis 20, vorzugsweise 10 bis 20, insbesondere 12 bis 18 Gew.% - bezogen auf das Gesamtgewicht des Konzentrats - enthalten.

Erfindungsgemäß ist das Verhältnis der Komponenten (A) zu (B) etwa 1 : (0,9-1,1), bevorzugt etwa 1 : etwa 1.

Die Emulsionskonzentrate können die Komponenten (A) und (B) insgesamt z. B. in Mengen von 2 bis 40, vorzugsweise 10 bis 40, insbesondere 15 bis 36 Gew.-% und besonders bevorzugt 20 bis 35 Gew.-%, bezogen auf das Gesamtgewicht des Konzentrats, enthalten.

### Komponente (C) - Polyole

Als Polyole werden mehrwertige Alkohole, d. h. organische Verbindungen, die mindestens 2 alkoholische Hydroxylgruppen im Molekül tragen, bezeichnet. In einer Ausführungsform der Erfindung enthalten die Polyole 2 bis 6 Hydroxylgruppen pro Molekül. In einer Ausführungsform der Erfindung werden als Polyole niederrnolekulare mehrwertige Alkohole eingesetzt, d.h. Verbindungen die 2 bis 18, insbesondere 2 bis 10, vorzugsweise 2 bis 6 C-Atome enthalten.

In einer bevorzugten Ausführungsform der Erfindung werden als Polyole (C) Verbindungen eingesetzt, die mindestens 2 Hydroxylgruppen pro Molekül tragen und aus 2 bis 18, vorzugsweise 2 bis 10, insbesondere aus 2 bis 6 C-Atomen bestehen.

In einer bevorzugten Ausführungsform der Erfindung werden als Polyole (C) Verbindungen eingesetzt, die 2 bis 6 Hydroxylgruppen pro Molekül tragen.

Besonders bevorzugt sind Polyole (C), die 2 bis 6 Hydroxylgruppen pro Molekül tragen und aus 2 bis 6 C-Atomen bestehen.

Als Polyole (C) können sowohl einzelne Polyole als auch Mischungen aus beliebigen Polyolen eingesetzt werden. In einer bevorzugten Ausführungsform werden als Polyole Mischungen aus mindestens 2, insbesondere mindestens 3 Polyolen eingesetzt.

Die Polyole (C) können noch weitere funktionelle Gruppen, insbesondere Aminogruppen, enthalten bzw. mit Stickstoff modifiziert sein. In einer bevorzugten Ausführungsform enthalten die Polyole außer den Hydroxylgruppen keine weiteren funktionellen Gruppen. Typische Beispiele für erfindungsgemäß einzusetzende Polyole sind:
- Glycerin, Diglycerin, Triglycerin, Tetraglycerin
- Alkylenglycole, wie beispielsweise Ethylenglycol, Diethylenglycol, Triethylenglykol, 1,2- Propylenglycol, 1 ,3-Propylenglycol, Butylenglycol, Hexylenglycol sowie Polyethylenglycole mit einem durchschnittlichen Molekulargewicht von 100 bis 1.000 Dalton;
- technische Oligogiyceringemische mit einem Eigenkondensationsgrad von 1,5 bis 10 wie etwa technische Diglyceringemische mit einem Diglyceringehalt von 40 bis 50 Gew.-%;
- Methyolverbindungen, wie Insbesondere Trimethylolethan, Trimethylolpropan, Trimethylolbutan, Pentaerythrit und Dipentaerythrit;
- kurzkettige Alkyglucoside, insbesondere solche mit 1 bis 8 Kohlenstoffen im Alkylrest, wie beispielsweise Methyl- und Butylglucosid;
- Zuckeralkohole mit 5 bis 12 Kohlenstoffatomen, wie beispielsweise Erythrit, Arabit, Adonit (Synonym Ribit), Xylit, Sorbit, Mannit und Dulcit (Synonym Galactit).
- Zucker mit 5 bis 12 Kohlenstoffatomen, wie beispielsweise Glucose oder Saccharose;
- Aminozucker, wie beispielsweise Glucamin;
- Dialkoholamine, wie Diethanolamin oder 2-Amino-1,3-propandiol.

In einer bevorzugten Ausführungsform der Erfindung wird als Polyol (C) mindestens eine Verbindung eingesetzt, ausgewählt aus der Gruppe, bestehend aus Glycerin, 1,2-Propylenglykol, Sorbit, Butylenglykol und Hexylenglykol.

Die Emulsionskonzentrate können die Komponente (C) In Mengen von 1 bis 20, vorzugsweise 10 bis 20, insbesondere 12 bis 18 Gew.% - bezogen auf das Gesamtgewicht des Konzentrats - enthalten.

Erfindungsgemäß beträgt das Gewichtsverhältnis der Komponenten (A), (B) und (C) etwa 1 : (0,9-1,1): (0,9 - 1,1), bevorzugt etwa 1 : etwa 1 : etwa 1. Dieses Gewichtsverhältnis sollte erfindungsgemäß eingehalten werden, um die gewünschten Wirkungen zu erzielen.

### Komponente (D) - Glyceride

Als Komponente (D) werden erfindungsgemäß Glyceride eingesetzt. Diese haben ebenfalls Coemulgatorwirkung. Mit Glyceriden sind erfindungsgemäß Glycerinester und Glycerinpartialester (Partialglyceride) gemeint.

Typische Beispiele für geeignete Partialglyceride sind Mono- und/oder Diglyceride von C12 bis C22 Fettsäuren mit Glycerin sowie deren technische Gemische. Beispielsweise seien genannt langkettige Hydroxyfeftsäuremonoglyceride, langkettige Hydroxyfettsäurediglyceride, Isostearinsäuremonoglycerid, Isostearinsäurediglycerid, Ölsäuremonoglycerid, Ölsäurediglycerid, Ricinolsäuremonoglycerid, Ricinolsäurediglycerid, Linolsäuremonoglycerid, Linolsäurediglycerid, Linolensäuremonoglycerid, Linolensäurediglycerid, Erucasäuremonoglycerid, Erucasäurediglycerid.

Typische Beispiele für geeignete Partialglyceride sind Mono- und/oder Diglyceride von Dicarbonsäuren mit 4 bis 8 C-Atomen mit Glycerin sowie deren technische Gemische. Besonders geeignet sind solche Partialglyeride, die einen Schmelzpunkt von >30°C aufweisen. Beispielsweise seien genannt Weinsäuremonoglycerid, Weinsäurediglycerid, Citronensäuremonoglycerid, Citronensäurediglycerid, Äpfelsäuremonoglycerid, Äpfelsäurediglycerid sowie deren technische Gemische, die untergeordnet aus dem Herstellungsprozess noch geringe Mengen an Triglycerid enthalten können.

Als lipohiler Coemulgator (D) sind erfindungsgemäß insbesondere Mono- und Diglyceride bzw. Mischungen dieser Partialglyceride einsetzbar. Zu den erfindungsgemäß einsetzbaren Glyceridgemischen zählen die von der Cognis GmbH vermarkteten Produkte Novata® AB und Novata® B (Gemisch aus C12-C18- Mono-, Di- und Triglyceriden) sowie Cutina® MD oder Cutina® GMS (Giycerylstearat).

Bevorzugt ist als Komponente (D) ein Glycerinoleat mit mehr als 80 Gew.-% ungesättigten C18-Fettsäuren enthalten, wobei der Monoesteranteil mehr als 90 Gew.-% beträgt.

Die Komponente (D) kann erfindungsgemäß z. B. in einem Anteil von etwa 1 bis 10 Gew.-%, bevorzugt etwa 2 bis 5 Gew.-%, bezogen auf das Emulsionskonzentrat, enthalten sein. Erfindungsgemäß ist im Verhältnis zur Komponente (A) im ein Gewichtsverhältnisbereich A : D von etwa 3 : 1 bis etwa 6 : 1 einzuhalten.

### Wässriger Anteil

Erfindungsgemäß ist ein Wassergehalt von 10 bis 20 Gew.-%, bezogen auf das Emulsionskonzentrat, einzuhalten.

### Weitere Bestandteile

Neben den genannten Bestandteilen können andere, in Emulsionskonzentraten übliche Bestandteile in üblichen Anteilen enthalten sein. Hierzu gehören z. B. Tenside, Konservierungsmittel, biogene Wirkstoffe, Verdickungsmittel, Überfettungsmiftel, Stabilisatoren, Polymere, Antioxidantien, Filmbildner, Quellmittel, Insektenrepellentien, Hydrotrope, Solubilisatoren, Parfümöle, Farbstoffe, Pigmente, UV-Filter etc.

Sollten weitere Bestandteile wasserunlöslich sein, sind sie der lipophilen Phase zuzurechnen. Je nach Polarität sind sie dann gegebenenfalls der Komponente (E) oder (F) zuzuordnen.

Die erfindungsgemäßen Emulsionskonzentrate enthalten die weiteren Bestandteile üblicherweise in Mengen von insgesamt < 25 Gew.%, insbesondere < 20 Gew.%, bezogen auf das Gesamtgewicht des Emulsionskonzentrates.

### Tenside

Tenside sind amphiphile Stoffe, die organische, unpolare Substanzen in Wasser lösen können. Sie sorgen, bedingt durch ihren spezifischen Molekülaufbau mit mindestens einem hydrophilen und einem hydrophoben Molekularteil, für eine Herabsetzung der Oberflächenspannung des Wassers, die Benetzung der Haut, die Erleichterung der Schmutzentfemung und -lösung, ein leichtes Abspülen und - je nach Wunsch- für Schaumregulierung.

Als Tenside werden üblicherweise oberflächenaktive Substanzen verstanden, die einen HLB-Wert von größer 20 aufweisen.

Als oberflächenaktive Stoffe können anionische, nichtionische, kationische und/oder amphotere bzw. zwitterionische Tenside enthalten sein. In tensidhaltigen kosmetischen Zubereitungen ist vorzugsweise wenigstens ein anionisches Tensid enthalten.

Die erfindungsgemäßen Zubereitungen können die Tenside üblicherweise In einer Menge von 0 bis 40 Gew.-%., vorzugsweise 0,05 bis 30 Gew.-% insbesondere 0,05 bis 20 Gew.-%, vorzugsweise 0,1 bis 15 Gew.-% und insbesondere 0,1 bis 10 Gew.-%, bezogen auf das Gesamtgewicht der Zubereitung, enthalten.

Typische Beispiele für **nichtionische Tenside** sind Fettalkoholpolyglycolether, Alkylphenolpolyglycolether, Fettsäurepolyglycolester, Fettsäureamidpolyglycolether, Fettamin-polyglycolether, alkoxylierte Triglyceride, Mischether bzw. Mischformale, gegebenenfalls partiell oxidierte Alk(en)yloligoglykoside bzw. Glucoronsäurederivate, Fettsäure-N-alkylglucamide, Proteinhydrolysate (insbesondere pflanzliche Produkte auf Weizenbasis), Polyolfettsäureester, Zuckerester, Sorbitanester, Polysorbate und Aminoxide. Sofern die nichtionischen Tenside Polyglycoletherketten enthalten, können diese eine konventionelle, vorzugsweise jedoch eine eingeengte Homologenverteilung aufweisen.

Als **zwitterionische Tenside** werden solche oberflächenaktiven Verbindungen bezeichnet, die im Molekül mindestens eine quartäre Ammoniumgruppe und mindestens eine -COO⁽⁻⁾- oder -SO₃⁽⁻⁾-Gruppe tragen. Besonders geeignete zwitterionische Tenside sind die so genannten Betaine wie die N-Alkyl-N,N-dimethylammoniumglycinate, beispielsweise das Kokosalkyldimethylammoniumglycinat, N-Acylaminopropyl-N,N-dimethylammoniumglycinate, beispielsweise das Kokosacylaminopropyldimethylammoniumglycinat, und 2-Alkyl-3-carboxylmethyl-3-hydroxyethylimidazolin mit jeweils 8 bis 18 C-Atomen in der Alkyl- oder Acylgruppe sowie das Kokosacylaminoethylhydroxyethylcarboxymethylglycinat. Ein bevorzugtes zwitterionisches Tensid ist das unter der INCI-Bezeichnung Cocamidopropyl Betaine bekannte Fettsäureamid-Derivat.

Ebenfalls, insbesondere als Co-Tenside geeignet, sind **ampholytische Tenside**. Unter ampholytischen Tensiden werden solche oberflächenaktiven Verbindungen verstanden, die außer einer C₈-C₁₈-Alkyl- oder Acylgruppe im Molekül mindestens eine freie Aminogruppe und mindestens eine -COOH- oder - SO₃H-Gruppe enthalten und zur Ausbildung innerer Salze befähigt sind. Beispiele für geeignete ampholytische Tenside sind N-Alkylglycine, N-Alkylpropionsäuren, N-Alkylaminobuttersäuren, N-Alkyliminodipropionsäuren, N-Hydroxyethyl-N-alkylamidopropylglycine, N-Alkyltaurine, N- Alkylsarcosine, 2-Alkylaminopropionsäuren und Alkylaminoessigsäuren mit jeweils etwa 8 bis 18 C-Atomen in der Alkylgruppe. Besonders bevorzugte ampholytische Tenside sind das N-Kokosalkylaminopropionat, das Kokosacylaminoethylaminopropionat und das C₁₂₋₁₈-Acylsaroosin.

Typische Beispiele für **amphotere bzw. zwitterionische Tenside** sind Alkylbetaine, Alkylamidobetaine, Aminopropionate, Aminoglycinate, Imidazoliniumbetaine und Sulfobetaine. Bei den genannten Tensiden handelt es sich ausschließlich um bekannte Verbindungen. Hinsichtlich Struktur und Herstellung dieser Stoffe sei auf einschlägige Übersichtsarbeiten auf diesem Gebiet verwiesen. Typische Beispiele für besonders geeignete milde, d.h. besonders hautverträgliche Tenside sind Feftalkoholpolyglycolethersulfate, Monoglyceridsulfate, Mono- und/oder Dialkylsulfosuccinate, Fettsäureisethionate, Fettsäuresarcosinate, Fettsäuretauride, Fettsäureglutamate, α-Olefinsulfonate, Ethercarbonsäuren, Alkylollgoglucoside und/ oder deren Gemische mit Alkyloligoglucosidcarboxylaten, Fettsäureglucamide, Alkylamidobetaine, Amphoacetale und/oder Proteinfettsäurekondensate, letztere vorzugsweise auf Basis von Weizenproteinen bzw. deren Salzen.

**Anionische Tenside** sind gekennzeichnet durch eine wasserlöslich machende, anionische Gruppe wie z. B. eine Carboxylat-, Sulfat-, Sulfonat oder Phosphat-Gruppe und einen lipophilen Rest. Hautverträgliche anionische Tenside sind dem Fachmann in großer Zahl aus einschlägigen Handbüchern bekannt und im Handel erhältlich. Es handelt sich dabei insbesondere um Alkylsulfate in Form ihrer Alkali-, Ammonium- oder Alkanolammoniumsalze, Alkylethersulfate, Alkylethercarboxylate, Acylisethionate, Acylsarkosinate, Acyltaurine mit linearen Alkyl- oder Acylgruppen mit 12 bis 18 C-Atomen sowie Sulfosuccinate und Acylglutamate in Form ihrer Alkali- oder Ammoniumsalze.

Typische Beispiele für **anionische Tenside** sind Seifen, Alkylbenzolsulfonate, Alkansulfonate, Olefinsulfonate, Alkylethersulfonate, Glycerinethersulfonate, α-Methylestersulfonate, Sulfofettsäuren, Alkylsulfate, Fettalkoholethersulfate, Glycerinethersulfate, Fettsäureethersulfate, Hydroxymischethersulfate, Monoglycerid(ether)sulfate, Fettsäureamid(ether)sulfate, Mono- und Dialkylsulfosuccinate, Mono- und Dialkylsulfosuccinamate, Suifotriglyceride, Amidseifen, Ethercarbonsäuren und deren Salze, Fettsäureisethionate, Fettsäuresarcosinate, Fettsäuretauride, N-Acylaminosäuren, wie beispielsweise Acyllactylate, Acyltartrate, Acylglutamate und Acylaspartate, Alkyloligoglucosidsulfate, Proteinfettsäurekondensate (insbesondere pflanzliche Produkte auf Weizenbasis) und Alkyl(ether)phosphate. Sofern die anionischen Tenside Polyglycoletherketten enthalten, können diese eine konventionelle, vorzugsweise jedoch eine eingeengte Homologenverteilung aufweisen.

Als **kationische Tenside** sind insbesondere quartäre Ammoniumverbindungen verwendbar. Bevorzugt sind Ammoniumhalogenide, insbesondere Chloride und Bromide, wie Alkyltrimethylammoniumchloride, Dialkyldimethylammoniumchloride und Trialkylmethylammoniumchloride, z. B. Cetyltrimethylammoniumchlorid, Stearyltrimethylammoniumchlorid, Distearyldimethylammoniumchlorid, Lauryldimethylammoniumchlorid, Lauryldimethylbenzylammoniumchlorid und Tricetylmethylammoniumchlorid. Weiterhin können die sehr gut biologisch abbaubaren quaternären Esterverbindungen, wie beispielsweise die unter dem Warenzeichen Stepantex^{®} vertriebenen Dialkylammoniummethosulfate und Methylhydroxyalkyldialkoyloxyalkylammoniummethosulfate und die entsprechenden Produkte der Dehyquart^{®}-Reihe, als kationische Tenside eingesetzt werden. Unter der Bezeichnung "Esterquats" werden im allgemeinen quaternierte Fettsäuretri-ethanolaminestersalze verstanden. Sie können den erfindungsgemäßen Zubereitungen einen besonderen Weichgriff verleihen. Es handelt sich dabei um bekannte Stoffe, die man nach den einschlägigen Methoden der organischen Chemie herstellt. Weitere erfindungsgemäß verwendbare kationische Tenside stellen die quaternisierten Proteinhydrolysate dar.

Als **Konservierungsmittel** eignen sich beispielsweise Phenoxyethanol, Ethylhexylglycerin, Dicaprylyl Glycol, Formaldehydlösung, Parabene, Pentandiol, Mischungen aus Phenoxyethanol und Ethylhexylglycerin (wie sie beispielsweise unter dem Handelsnamen Euxyl PE 9010 erhältlich sind), oder Sorbinsäure sowie die unter der Bezeichnung Surfacine® bekannten Silberkomplexe und die in Anlage 6, Teil A und B der Kosmetikverordnung aufgeführten weiteren Stoffklassen.

In einer bevorzugten Ausführungsform der Erfindung wird das Konservierungsmittel ausgewählt aus der Gruppe bestehend aus Phenoxyethanol, Formaldehydlösung, Parabenen, organische Säuren und Mischungen daraus, gegebenenfalls in Kombination mit Pentandiol und/oder Ethylhexylglycerin bzw. Octandiol und Ethylhexyiglycerin (Handelsname Sensiva SC 10).

In einer bevorzugten Ausführungsform der Erfindung enthalten die erfindungsgemäßen Emulsionskonzentrate als biogenen Wirkstoff mindestens eine Verbindung ausgewählt aus Vitaminen, Allantoin, Bisabolol und Pflanzenextrakten.

In einer bevorzugten Ausführungsform der Erfindung enthalten die erfindungsgemäßen Emulsionskonzentrate als biogenen Wirkstoff mindestens eine Verbindung ausgewählt aus Tocopherol, Tocopherolacetat, Tocopherolpalmitat, Ascorbinsäure, (Desoxy)Ribonucleinsäuren und deren Fragmentierungsprodukten, β-Glucanen, Retinol, Bisabolol, Allantoin, Phytantriol, Panthenol, AHA-Säuren, Aminosäuren, Ceramiden, Pseudoceramiden, essentiellen Ölen, Pflanzenextrakten, wie z. B. Aloe vera, Prunusextrakt, Bambaranussextrakt und Vitaminkomplexe und Mischungen daraus.

In einer Ausführungsform der Erfindung enthalten die erfindungsgemäßen Emulsionskonzentrate als weiteren Bestandteil mindestens ein Verdickungsmittel.

Als Verdickungsmittel eignen sich beispielsweise Aerosil-Typen (hydrophile Kieselsäuren), Polysaccharide, insbesondere Xanthan-Gum, Guar-Guar, Agar-Agar, Alginate und Tylosen, Carboxymethylcellulose und Hydroxyethyl- und Hydroxypropylcellulose, Polyvinylalkohol, Polyvinylpyrrolidon und Bentonite wie z. B. Bentone® Gel VS-5PC (Rheox).

Als Insekten-Repellenfien kommen beispielsweise N,N-Diethyl-m-toluamid,1 ,2-Pentandiol oder 3-(N-n-Butyl-N-acetyl-amino)-propionsäureethylester), welches unter der Bezeichnung Insect Repellent® 3535 von der Merck KGaA vertrieben wird, sowie Butylacetylaminopropionate in Frage.

Als Parfümöle seien genannt Gemische aus natürlichen und synthetischen Riechstoffen. Natürliche Riechstoffe sind Extrakte von Blüten, Stengeln und Blättern, Früchten, Fruchtschalen, Wurzeln, Hölzern, Kräutern und Gräsem, Nadeln und Zweigen, Harzen und Balsamen. Weiterhin kommen tierische Rohstoffe, wie beispielsweise Zibet und Castoreum sowie synthetische Riechstoffverbindungen vom Typ der Ester, Ether, Aldehyde, Ketone, Alkohole und Kohlenwasserstoffe in Frage.

Der Begriff **Pigment** umfasst Partikel jeder Form, die weiß oder gefärbt, organisch oder anorganisch sind, in den Zubereitungen nicht löslich sind, und dem Zweck dienen, die Zubereitung zu färben.

In einer bevorzugten Ausführungsform werden anorganische Pigmente verwendet, besonders bevorzugt sind Metalloxide.

Als **anorganische Pigmente** seien exemplarisch genannt: Titandioxid, optional oberflächenbeschichtet, Zirkonium oder Ceriumoxide und Zink-, Eisen- (Schwarz, Gelb oder Rot) und Chromoxide, Manganviolett, Ultramarine Blau, Chromhydrate und Eisen(III)blau, Metallpulver wie Aluminiumpulver oder Kupferpulver.
In einer bevorzugten Ausführungsform der Erfindung ist das Pigment ausgewählt aus den anorganischen Pigmenten, vorzugsweise aus den Metalloxiden. In einer bevorzugten Ausführungsform ist das Pigment ausgewählt aus der Gruppe bestehend aus Titandioxid, Zinkoxid, Eisenoxid und Mischungen daraus.
Die Pigmente können sowohl einzeln als auch in Mischungen vorliegen.
Bevorzugt im Sinne der vorliegenden Erfindung sind Pigmentmischungen aus Weiß-Pigmenten (z. B. Kaolin, Titandioxid oder Zinkoxid) und anorganischen Farbpigmenten (z. B. Eisenoxid Pigmente, Chromoxide), wobei die Pigmente beschichtet ("gecoatet") oder unbeschichtet vorliegen können. Unter den Farbpigmenten sind Eisenoxide besonders bevorzugt.

Vorteilhaft im Sinne der vorliegenden Erfindung können das oder die Pigmente auch aus der Gruppe der Effektpigmente gewählt werden, welche der kosmetischen Zubereitung neben der reinen Farbe eine zusätzliche Eigenschaft - wie z. B. eine Winkelabhängigkeit der Farbe (changieren, Flop), Glanz (nicht Oberflächenglanz) oder Textur - verleihen. Solche Effektpigmente werden erfindungsgemäß vorteilhaft zusätzlich zu einem oder mehreren Weiß- und/oder Farbpigmenten eingesetzt.

Die bedeutendste Gruppe der Effektpigmente stellen die Glanzpigmente dar, zu denen nach DIN 55944: 2003-11 die Metalleffektpigmente und die Perlglanzpigmente gehören. Einige spezielle Effektpigmente lassen sich diesen beiden Gruppen nicht zuordnen, z. B. plättchenfoermiges Graphit, plättchenförmiges Eisenoxid und mikronisiertes Titandioxid, wobei mikronisiertes Titandioxid keinen Glanzeffekt, sondern einen winkelabhängigen Lichtstreueffekt erzeugt. Bei den Glanzpigmenten nach DIN 55943: 2001-10 handelt es sich vorwiegend um plättchenfoermige Effektpigmente. Parallel orientiert zeigen Glanzpigmente einen charakteristischen Glanz. Die optische Wirkung von Glanzpigmenten beruht auf der gerichteten Reflexion an metallischen Teilchen (Metalleffektpigmente), an transparenten Teilchen mit hoher Brechzahl (Perlglanzpigmente) oder auf dem Phaenomen der Interferenz (Interferenzpigmente) (DIN 55944: 2003-11).

Beispiele für erfindungsgemäß bevorzugte handelsübliche Effektpigmente sind: Timiron and #174; von Merck, Iriodin and #174; von Merck (Perl- und Farbglanzpigmente für dekorative technische Anwendungen), Xirallic and #174; von Merck (farbintensive Kristalleffektpigmente).

Ferner können die erfindungsgemäßen Zubereitungen vorteilhaft auch **organische Farbpigmente** enthalten, d. h. organische Farbstoffe, welche in der Zubereitung praktisch unlöslich sind. Nach **DIN 55944: 1990-04** können organische Pigmente nach chemischen Gesichtspunkten in Azopigmente und polycyclische Pigmente sowie nach farblichen Gesichtspunkten in Bunt- oder Schwarzpigmente eingeteilt werden. Organische Weißpigmente sind ohne praktische Bedeutung.
Die Pigmente können vorteilhaft im Sinne der vorliegenden Erfindung auch in Form kommerziell erhältlicher öliger oder wässriger Vordispersionen zur Anwendung kommen.

Es ist ferner möglich, dass die erfindungsgemäße Zubereitung einen oder mehrere **Farbstoffe** enthält.

Die Farbstoffe können sowohl synthetischen als auch natürlichen Ursprungs sein.
Eine Liste von geeigneten Farbstoffen findet sich in EP 1 371 359 A2, S.8, Z. 25-57, S.9 und S.10 sowie S.11, Z. 1 bis 54, auf welche hiermit explizit Bezug genommen wird.

Als Farbstoffe und Pigmente eignen sich insbesondere die gemäß Annex IV der Kommissions Direktive zugelassenen Farbstoffe und Pigmente (in der Fassung: Commission Directive 2007/22/EC of 17 April 2007 amending Council Directive 76/768/EEC, concerning cosmetic products, for the purposes of adapting Annexes IV and VI thereto to technical progress), auf die hiermit explizit Bezug genommen wird.

Erfindungsgemäß sind als **UV-Lichtschutzfilter** bei Raumtemperatur flüssige oder kristalline organische Substanzen (Lichtschutzfilter) geeignet, die in der Lage sind, ultraviolette Strahlen zu absorbieren und die aufgenommene Energie in Form längerwelliger Strahlung, z.B. Wärme wieder abzugeben. UV-Filter können öllöslich oder wasserlöslich sein. Als typische öllösliche UV-B-Filter bzw. Breitspektrum-UV A/B-Filter sind z.B. zu nennen:
➢ 3-Benzylidencampher bzw. 3-Benzylidennorcampher (Mexoryl SDS 20) und dessen Derivate, z.B. 3-(4-Methylbenzyliden)campher wie in der EP 0693471 B1 beschrieben ➢ 3-(4'-Trimethylammonium) benzyliden- boman-2-on-methylsulfat (Mexoryl SO) ➢ 3,3'-(1,4-Phenylendimethin)-bis (7,7-dimethyl-2-oxobicyclo-[2.2.1] heptan-1-methansulfonsäure) and salts (Mexoryl SX)
➢ 3-(4'-Sulfo)-benzyliden-boman-2-on and salts (Mexoryl SL)
➢ Polymer von N-[2(und 4)- (2-oxoborn-3-ylidenmethyl)benzyl]acrylamid Mexoryl SW) ➢ 2-(2H-Benzotriazol-2-yl)-4-methyl-6-(2-methyl-3-(1,3,3,3-tetramethyl-1-(trimethylsilyloxy)disiloxanyl)propyl) phenol (Mexoryl XL)
➢ 4-Aminobenzoesäurederivate, vorzugsweise 4-(Dimethylamino)benzoesäure-2-ethyl-hexylester, 4-(Dimethylamino)benzoesäure-2-octylester und 4-(Dimethylamino)benzoe-säureamylester,
➢ Ester der Zimtsäure, vorzugsweise 4-Methoxyzimtsäure-2-ethylhexylester, 4-Methoxy-zimtsäurepropylester, 4-Methoxyzimtsäureisoamylester, 2-Cyano-3,3-phenyliimtsäure-2-ethylhexylester (Octocrylene);
➢ Ester der Salicylsäure, vorzugsweise Salicylsäure-2-ethylhexylester, Salicylsäure-4-iso-propylbenzylester, Salicylsäurehomomenthylester;
➢ Derivate des Benzophenons, vorzugsweise 2-Hydroxy-4-methoxybenzophenon, 2-Hydroxy-4-methoxy-4'-methylbenzophenon, 2,2'-Dihydroxy-4-methoxybenzophenon;
➢ Ester der Benzalmalonsäure, vorzugsweise 4-Methoxybenzmalonsäuredi-2-ethylhexyl-ester;
➢ Triazinderivate, wie z.B. 2,4,6-Trianilino-(p-carbo-2'-ethyl-1'-hexyloxy)-1,3,5-triazin und 2,4,6-Tris[p-(2-ethylhexyl-oxycar-bonyl) anilino]-1,3,5-triazin (Uvinul T 150) wie in der EP 0818450 A1 beschrieben oder 4,4'-[(6-[4-((1,1-Dimethylethyl)amino-carbonyl) phenyl-amino]-1,3,5-triazin-2,4-diyl)dümino] bis(benzoesäure-2- ethylhexylester) (Uvasorb® HEB):
➢ 2,2(-Methylen-bis(6-(2H-benzotriazol-2-yl)-4- (1,1,3,3-tetramethyl-butyl)phenol) (Tinosorb M);
➢ 2,4-Bis[4-(2-ethylhexyloxy)-2- hydroxyphenyl]-6-(4- methoxyphenyl)-1,3,5- triazin (Tinosorb S);
➢ Propan-1,3-dione, wie z.B. 1-(4-tert.Butylphenyl)-3-(4'methoxyphenyl)propan-1,3-dion;
➢ Ketotricyclo(5.2.1.0)decan-Derivate, wie in der EP 0694521 B1 beschrieben;
➢ Dimethicodiethylbenzalmalonate (Parsol SLX).

Als wasserlösliche UV - Filter kommen in Frage:
➢ 2-Phenylbenzimidazol-5-sulfonsäure und deren Alkali-, Erdalkali-, Ammonium-, Alkylammonium-, Alkanolammonium- und Glucammoniumsalze;
➢ 2,2(-(1,4-Phenylen)bis(1H-benzimidazol- 4,6-disulfon-säure, Mononatriumsalz) (Neo Heliopan AP)
➢ Sulfonsäurederivate von Benzophenonen, vorzugsweise 2-Hydroxy-4-methoxybenzo-phenon-5-sulfonsäure und ihre Salze;
➢ Sulfonsäurederivate des 3-Benzylidencamphers, wie z.B. 4-(2-Oxo-3-bomylidenmethyl)-benzolsulfonsäure und 2-Mothyl-5-(2-oxo-3-bornyliden)sulfonsäure und deren Salze.

Als typische UV-A-FÜter kommen insbesondere Derivate des Benzoylmethans in Frage, wie beispielsweise 1-(4'-tert.Butylphenyl)-3-(4'-methoxyphenyl)propan-1,3-dion, 4-tert.-Butyl-4'-methoxydibenzoylmethan (Parsol® 1789), 1-Phenyl-3-(4'-Isopropylphenyl)-propan-1,3-dion sowie Enaminverbindungen, wie beschrieben in der DE 19712033 A1 (BASF) sowie Benzoic Acid, 2-[4-(Diethylamino)-2-Hydroxybenzoyl]-, Hexyl Ester (Uvinul® A plus).

Die UV-A und UV-B-Filter können selbstverständlich auch in Mischungen eingesetzt werden. Besonders günstige Kombinationen bestehen aus den Derivaten des Benzoylmethans, z.B. 4-tert.-Butyl-4'-methoxydibenzoylmethan (Parsol® 1789) und 2-Cyano-3,3-phenylzimtsäure-2-ethyl-hexylester (Octocrylene) in Kombination mit Estern der Zimtsäure, vorzugsweise 4-Methoxyzimtsäure-2-ethylhexylester und/oder 4-Methoxyzimtsäurepropylester und/oder 4-Methoxyzimtsäureisoamylester. Vorteilhaft werden derartige Kombinationen mit wasserlöslichen Filtern wie z.B. 2-Phenylbenzimidazol-5-sulfonsäure und deren Alkalt-, Erdalkali-, Ammonium-, Alkylammonium-, Alkanolammonium- und Glucammoniumsalze kombiniert.

Als UV-Lichtschutzfilter eignen sich insbesondere die gemäß Annex VII der Kommissions Direktive (in der Fassung **Commission Directive 2005/9/EC of 28 January 2005 amending Council Directive 7617681EEC**, concerning cosmetic products, for the purposes of adapting Annexes VII thereof to technical progress) zugelassen Stoffe, auf die hier explizit Bezug genommen wird.

Neben den genannten löslichen Stoffen kommen für diesen Zweck auch unlösliche Lichtschutzpigmente, nämlich feindisperse Metalloxide bzw. Salze in Frage. Beispiele für geeignete Metalloxide sind insbesondere Zinkoxid und Titandioxid und daneben Oxide des Eisens, Zirkoniums, Siliciums, Mangans, Aluminiums und Cers sowie deren Gemische. Als Salze können Silicate (Talk), Bariumsulfat oder Zinkstearat eingesetzt werden. Die Oxide und Salze werden in Form der Pigmente für hautpflegende und hautschützende Emulsionen und auch für die dekorative Kosmetik verwendet. Die Partikel sollten einen mittleren Durchmesser von weniger als 100 nm, vorzugsweise zwischen 5 und 50 nm und insbesondere zwischen 15 und 30 nm aufweisen. Sie können eine sphärische Form aufweisen, es können jedoch auch solche Partikel zum Einsatz kommen, die eine ellipsoide oder in sonstiger Weise von der sphärischen Gestalt abweichende Form besitzen. Die Pigmente können auch oberflächenbehandelt, d.h. hydrophilisiert oder hydrophobiert vorliegen. Typische Beispiele sind gecoatete Titandioxide, wie z.B. Titandioxid T 805 (Degussa) oder Eusolex® T, Eusolex® T-2000, Eusolex® T-Aqua, Eusolex® AVO, Eusolex® T-ECO, Eusolex® T-OLEO und Eusolex® T-S (Merck). Typische Beispiele für sind Zinkoxide, wie z.B. Zinc Oxide neutral, Zinc Oxide NDM (Symrise) oder Z-Cote® (BASF) oder SUNZnO-AS und SUNZnO-NAS (Sunjun Chemical Co. Ltd.). Als hydrophobe Coatingmittel kommen dabei vor allem Silicone und dabei speziell Trialkoxyoctylsilane oder Simethicone in Frage. In Sonnenschutzmitteln werden bevorzugt sogenannte Mikro- oder Nanopigmente eingesetzt. Vorzugsweise wird mikronisiertes Zinkoxid verwendet. Weitere geeignete UV-Lichtschutzfilter sind der Übersicht von P. Finkel in SÖFW-Journal 122, 811996, S. 543.548 sowie Parf. Kosm. 80. Jahrgang, Nr. 3/1999, S.10 bis 16 zu entnehmen.

Neben den beiden vorgenannten Gruppen primärer Lichtschutzstoffe können auch sekundäre Lichtschutzmittel vom Typ der **Antioxidantien** eingesetzt werden, die die photochemische Reaktionskette unterbrechen, welche ausgelöst wird, wenn UV-Strahlung in die Haut eindringt. Typische Beispiele hierfür sind Aminosäuren (z.B. Glycin, Histidin, Tyrosin, Tryptophan) und deren Derivate, Imidazole (z.B. Urocaninsäure) und deren Derivate, Peptide wie D,L-Camosin, D-Camosin, L-Camosin und deren Derivate (z.B. Anserin), Carotinoide, Carotine (z.B. -Carotin, -Carotin, Lycopin) und deren Derivate, Chlorogensäure und deren Derivate, Liponsäure und deren Derivate (z.B. Dihydroliponsäure), Auro-thioglucose, Propylthlouracil und andere Thiole (z.B. Thioredoxin, Glutathion, Cystein, Cystin, Cysta-min und deren Glycosyl-, N-Acetyl-, Methyl-, Ethyl-, Propyl-, Amyl-, Butyl- und Lauryl-, Palmitoyl-, Oleyl-, -Linoleyl-, Cholesteryl- und Glycerylester) sowie deren Salze, Dilaurylthiodipropionat, Distearylthiodipropionat, Thiodipropionsäure und deren Derivate (Ester, Ether, Peptide, Lipide, Nukleotide, Nukleoside und Salze) sowie Sulfoximinverbindungen (z.B. Buthioninsulfoximine, Homocysteinsulfoximin, Butioninsulfone, Penta-, Hexa-, Heptathioninsulfoximin) in sehr geringen verträglichen Dosierungen (z.B. pmol bis mol/kg), ferner (Metall)-Chelatoren (z.B. a-Hydroxyfettsäuren, Palmitinsäure, Phytinsäure, Lactoferrin), α-Hydroxysäuren (z.B. Citronensäure, Milchsäure, Äpfelsäure), Huminsäure, Gallensäure, Gallenextrakte, Bilirubin, Biliverdin, EDTA, EGTA und deren Derivate, ungesättigte Fettsäuren und deren Derivate (z.B. gamma-Linolensäure, Linolsäure, Ölsäure), Folsäure und deren Derivate, Ubichinon und Ubichinol und deren Derivate, Vitamin C und Derivate (z.B. Ascorbylpalmitat, Mg-Ascor-bylphosphat, Ascorbylacetat), Tocopherole und Derivate (z.B. Vitamin-E-acetat), Vitamin A und Derivate (Vitamin-A-palmitat) sowie Koniferylbenzoat des Benzoeharzes, Rutinsäure und deren Derivate, a-Glycosylrutin, Ferulasäure, Furfurylidenglucitol, Camosin, Butylhydroxytoluol, Butylhydroxyanisol, Nordihydroguajakharzsäure, Nordihydroguajaretsäure, Trihydroxybutyrophenon, Harnsäure und deren Derivate, Mannose und deren Derivate, Superoxid-Dismutase, Zink und dessen Derivate (z.B. ZnO, ZnSO4) Selen und dessen Derivate (z.B. Selen-Methionin), Stilbene und deren Derivate (z.B. Stilbenoxid, trans-Stilbenoxid) und die erfindungsgemäß geeigneten Derivate (Salze, Ester, Ether, Zucker, Nukleotide, Nukleoside, Peptide und Lipide) dieser genannten Wirkstoffe.

In einer Ausführungsform der Erfindung enthaltend die erfindungsgemässen Emulsionskonzentrate als weiteren Bestandteil mindestens ein desodorierenden Wirkstoff.

Desodorierende Wirkstoffe wirken Körpergerüchen entgegen, überdecken oder beseitigen sie. Körpergerüche entstehen durch die Einwirkung von Hautbakterien auf apokrinen Schweiss, wobei unangenehm riechende Abbauprodukte gebildet werden. Dementsprechend eignen sich als deosodorierende Wirkstoffe u.a. keimhemmende Mittel, Enzyminhibitoren, Geruchsabsorber oder Gerüchsüberdecker.

Als Stabilisatoren können Metallsalze von Fettsäuren, wie z. B. Magnesium-, Aluminium- und/oder Zinkstearat bzw. -ricinoleat eingesetzt werden.

Die weiteren Bestandteile der Zubereitungen (wie z.B. Konservierungsmittel, kosmetische Wirkstoffe wie z.B. DHA- Dihydroxyaceton -, UV-Filter etc.) werden je nach ihrer Löslichkeit entweder über die Wasserphase oder über die lipophile Phase zugegeben.

### Herstellung der erfindunosoemäßen Emulsionskonzentrate

Eine Möglichkeit der Herstellung der erfindungsgemäßen Emulsionskonzentraten ist wie folgt: Alkyl- und/oder Alkenyloligoglykoside (A), Polyglycerolester (B) zusammen mit dem Polyol (C), Glycerid (D), wasserunlöslicher Komponente mit einer Polarität von etwa 30 - 40 mN/m (E), wasserunlöslicher Komponente mit einer Polarität von etwa 15- 25 mN/m (F) und Wasser unter Rühren auf 70 - 75°C für 60 min. erhitzen. Anschließend unter Rühren auf 30°C abkühlen und ggf. den pH-Wert einstellen auf pH 5,0-6,0.

### Verwendung der Emulsionskonzentrate

Die erfindungemäßen Emulsionskonzentrate können direkt als kosmetische oder pharmazeutische Zubereitung verwendet werden. Ein weiterer Gegenstand der Erfindung betrifft daher die Verwendung der erfindungsgemäßen Emulsionskonzentrate als kosmetische oder pharmazeutische Zubereitungen.

Die erfindungsgemäßen Emulsionskonzentrate sind feinteilig und sehr lagerstabil, sie eignen sich daher ganz besonders als vorgefertigter Emulsionsbaustein, der aufgrund seiner Fliessfähigkeit gut für die Lagerung und den Transport an einen Verarbeitungsort mit geringer technischer Ausrüstung geeignet ist, um dort mit einfachsten Mitteln brauchbare kosmetische und pharmazeutische Emulsionen, insbesondere Öl-in-Wasser Emulsionen herzustellen.

Ein Gegenstand der Erfindung betrifft demnach die Verwendung der erfindungsgemäßen Emulsionskonzentrate zur Herstellung von kosmetischen oder pharmazeutischen Zubereitungen.

Das erfindungsgemäße Emulsionskonzentrat eignet sich insbesondere zur Herstellung kosmetischer oder pharmazeutischer Öl-in-Wasser-Emulsionen. Dabei kann entweder die kontinuierliche wässrige Phase oder die lipophile Phase oder beides ohne weitere Wärmezufuhr in das Emulslonskonzentrat eingearbeitet werden.

Die wässrige Phase, mit der das Emulsionskonzentrat verdünnt wird, kann beliebige wasserlösliche Bestandteile, z.B. wasserlösliche kosmetische Wirkstoffe, wasserlösliche Proteine oder Proteinabbauprodukte, Konservierungsmittel, Farbstoffe, Duftstoffe, Magnesiumsalze oder andere übliche wasserlösliche Komponenten gelöst enthalten. Bevorzugt enthält die wässrige, kontinuierliche Phase ein wasserlösliches, natürliches oder synthetisches Polymerisat, das die kosmetischen Eigenschaften der Emulsionen durch Erhöhung der Viskosität verbessert. Eine besonders wirksame Kombination von Hydrocolloiden zur Verbesserung der kosmetischen Eigenschaften solcher Emulsionen ist ein Gemisch aus nichtionischen Celluloseethern, z.B. Hydroxypropylcellulose und vernetzten Acrytsäure-Poiymerisaten, wie sie z.B. unter der Handelsbezeichnung Carbopol® erhältlich sind (vgl. DE 3521713 A1). Darüber hinaus sind besonders geeignete Polymere unter den Handelsbezeichnungen (Cosmedia^{®} ATH, Cosmedia^{®} SP, Cosmedia^{®} CHT (E), Ultragel^{™} 300, Rheccare^{™} TTA, Rheocare^{™} TTN, Rheocare^{™} TTN-2) bekannt.

Die liphophile Phase, mit der das Emulsionskonzentrat verdünnt wird, kann beliebige lipophile Bestandteile, z.B. liphophile kosmetische Wirkstoffe, enthalten. Als lipophile Phase eignen sich alle als Komponente (E) und (F) geeigneten Verbindungen.

In der Regel werden 1 bis 50, bevorzugt 2 bis 30, insbesondere 4 bis 10 Gew.-% des Emulsionskonzentrates für die Herstellung der kosmetischen oder pharmazeutischen Zubereitungen eingesetzt.

### Kosmetische Zubereitungen

Ein weiterer Gegenstand der Erfindung betrifft kosmetische oder pharmazeutische Zubereitungen, welche das erfindungsgemäße Emulsionskonzentrat enthalten.

Überraschenderweise wurde gefunden, dass durch die Auswahl der Komponenten sowie die erfindungsgemäßen Verhältnisse der Komponenten zueinander stabile, feinteilige Zubereitungen, insbesondere Öl-in-Wasser Emulsionen erhalten werden können, die ohne ethoxylierte Substanzen formuliert werden können.

Die erfindungsgemäßen Zubereitungen können beispielsweise hergestellt werden, indem man die erfindungsgemäßen Emulsionskonzentrate entsprechend verdünnt. Im einfachsten Fall erfolgt die Verdünnung mit Wasser, da so die erfindungsgemäßen Verhältnisse der Komponenten nicht verändert werden.

### Verwerdung zur Beschichtung von Substraten und beschichtete Substrate

Die erfindungsgemäßen Emulsionskonzentrate sowie die erfindungsgemäßen kosmetischen oder pharmazeutischen Zubereitungen eignen sich insbesondere zur Beschichtung von Substraten.

Die Emulsionskonzentrate sowie die Zubereitungen sind besonders geeignet zur Applikation auf Papieren, Tüchern, Textilien und Watteprodukten, die im Bereich der Babypflege und -hygiene sowie im Bereich der Make-up-Entfemung, insbesondere der Augen-Make-up-Entfemung, im Bereich der Damenhygiene (Tampons, Damenbinden, Slip-Einlagen) und im Bereich der Körperhygiene (Toilettenpapier, Feuchttoilettenpapier) eingesetzt werden.

Ein weiter Gegenstand der Anmeldung ist daher die Verwendung der erfindungsgemäßen Emulsionskonzentrate oder der erfindungsgemäßen Zubereitungen auf Papieren, Vliesen (Nonwoven) und Geweben (Woven). Erfindungsgemäß zählen hierzu alle Papierarten, Vliese und Gewebe, die dem Fachmann geläufig sind, und Produkte, die daraus herstellbar sind, wie z. B. Toilettenpapier, Papiertaschentücher, Tissues, Wipes, Watte, Wattepads, Make-up Entferner, Tampons, Binden, SlipEinlagen, Windeln, Babypflegetücher, Babyreinigungstücher, Textilien, etc.

Ebenso Gegenstand der Erfindung sind Papier-, Vlies- und Gewebe-Produkte zur Körperpflege und - reinigung, welche das erfindungsgemäße Emulsionskonzentrat so oder in verdünnter Form enthalten.

Als Substrate dieser Papier-, Vlies- und Gewebe-Produkte seinen exemplarisch genannt: Träger aus Textilfaser, z.B. aus Naturfaser, wie Cellulose, Seide, Wolle, Regeneratcellulose (Viskose, Rayon), Cellulosederivate und/oder synthetische Fasern, wie z. B.Polyester-, Polypropylen-, Polyethylenterephtalat-, Polyamin-, Polyolifin-, Polyacrylnitril-Fasern oder Mischungen solcher Fasern, gewebt oder ungewebt.

Die erfindungsgemäßen Produkte können nach dem Fachmann bekannten Verfahren hergestellt werden. Der Auftrag der erfindungsgemäßen Emulsionskonzentrate oder der erfindungsgemäßen Zubereitungen auf die erfindungsgemäßen Papier-, Vlies- und Gewebe-Produkte zur Körperpflege und - reinigung erfolgt dabei nach dem Fachmann bekannten Methoden, wie beispielsweise Imprägnieren, Tränken, Eintauchen, Ansprühen, Abstreifen oder Beschichten. Dies kann sowohl bei Raumtemperatur als auch bei erhöhten Temperaturen erfolgen.

Die erfindungsgemäßen Emulsionskonzentrate oder die erfindungsgemäßen Zubereitungen können vor dem Auftrag auf das Papier-, Vlies- und Gewebe-Substrat verdünnt werden und gegebenenfalls kann das erhaltene Papier-, Vlies- und Gewebe-Produkt anschließend getrocknet werden.

### Beispiele

Die folgenden Beispiele sollen die vorliegende Erfindung näher erläutern.

Die in Tab. 1 aufgeführten Emulsionskonzentrate wurden wie folgt hergestellt.
Alkyl- und/oder Alkenyloligoglykoside (A), Polyglycerolester (B) zusammen mit dem Polyol (C), Glycerid (D), wasserunlöslicher Komponente mit einer Polarität von etwa 30 - 40 mN/m (E), wasserunlöslicher Komponente mit einer Polarität von etwa 15-25 mN/m(F) und Wasser wurden unter Rühren auf 70 - 75°C für 60 min. erhitzt. Anschließend wurde unter Rühren auf 30°C abgekühlt und ggf. der pH-Wert auf pH 5,0-6,0 eingestellt.

Tabelle 1 zeigt die Zusammensetzung der Emulsionskonzentrate und deren Viskositäten.
Die Viskosität wurde sowohl bei Raumtemperatur (RT = etwa 23°C) als auch bei 15°C mit einem Brookfield Rotationsviskosimeter (Typ RVF, Spindel 5,10 UpM) bestimmt.
Es zeigte sich, dass sowohl eine gute Lagerstabilität als auch gute Viskositätseigenschaften, sowohl bei Raumtemperatur als auch bei niedriger Temperatur, bei Einhaltung der erfindungsgemäßen Parameter, erhalten wurde.
Des Weiteren sind die Stabilitätsergebnisse der Verdünnungen mit Wasser sowie deren Teilchengröße dargestellt.
Nur die erfindungsgemäßen Verdünnungen der Emulsionskonzentrate sind bei Lagerung stabil.

**Tab. 1: Zusammensetzung und Ergebnisse erfindungsgemäßer Emulsionskonzentrate und von Vergleichsemulsionskonzentraten (alle Angaben in Gew.-% bezogen auf das Gewicht des Konzentrats)**

| | **Komponente INCI** | **Bsp.1** | **Bsp. 2** | **Bsp. 3** | **Bsp. 4** | **V.1** | **V. 2** | **V. 3** | **V. 4** |
|---|---|---|---|---|---|---|---|---|---|
| (A) | Lauryl Glucoside | 15,0 | 13,75 | 16,25 | 13,75 | 12,5 | 16,25 | 5,75 | 22,0 |
| (B) | Polyglyceryl-2-Dipolyhydroxy-stearat | 15,0 | 13,75 | 16,25 | 13,75 | 12,5 | 16,25 | 5,75 | 22,0 |
| (C) | Glycerin | 15,0 | 13,75 | 16,25 | 13,75 | 12,5 | 21,25 | 5,75 | 22,0 |
| (D) | Glyceryl Oleate | 3,0 | 3,0 | 3,0 | 3,0 | 3,0 | 3,0 | - | 3,8 |
| (E) | Dicaprylyl Ether | 13,0 | 13,0 | - | - | 8,0 | - | - | 4,1 |
| | Cetearyl Alcohol | - | - | - | - | - | - | 4,5 | - |
| (E) | Dicaprylyl Carbonate | - | - | 8,0 | - | - | 3,0 | 34,75 | - |
| (E) | Propylheptyl Caprylate | - | - | - | 13,0 | - | - | - | - |
| (F) | Caprylic/Capric Triglyceride | 24,0 | 29,0 | 24,0 | 26,0 | 34,0 | - | 37,5 | 4,1 |
| (F) | Cocoglycerides | - | - | - | 3,0 | - | - | - | - |
| (F) | Olus | - | - | - | - | 5,0 | 24,0 | - | - |
| | Citric Acid (zur PH-Einstellung) | | | | | | | | |
| | Wasser | 15,0 | 13,75 | 16,25 | 13,75 | 12,5 | 16,25 | 6,0 | 22,0 |
| | Viskosität (RT), mPa*s | 3600 | 2000 | 6400 | 1200 | 3200 | 23200 | Konzentrat, instabil | 4800 |
| | Viskosität (15°C),mPa*s | 6400 | 4800 | 14000 | 10000 | 7200 | 55000 - | | 8800 |
| | Teilchengröße, 5% Verdünnung Mittelwert (Coulter LS),nm | 81 | 82 | 79 | 83 | 304 | 203 | - | 208 |
| | Aussehen 5 % wässrige Verdünnung | transp. | transp. | transp. | transp. | trüb | trüb | - | trüb |

Tabellen 2 bis 4 zeigen die Verhältnisse der Komponenten zueinander.

Tabelle 5 gibt wässrige Verdünnungen der Emulsionskonzentrate 1 bis 4 wieder.

**Tab. 2: Gewichtsverhältnisse der Komponenten (A), (B) und (C) und ggf. Wasser**

| Beispiel | Verhältnis (A) : (B): (C) |
|---|---|
| Beispiel 1 | 1:1:1 |
| Beispiel 2 | 1:1:1 |
| Beispiel 3 | 1:1:1 |
| Beispiel 4 | 1:1:1 |
| Vergleichsbeispiel 1 | 1:1:1 |
| Vergleichsbeispiel 2 | 1:1:1,3 |
| Vergleichsbeispiel 3 | 1:1:1, jedoch Wasser <10 Gew.-% |
| Vergleichsbeispiel 4 | 1:1:1, jedoch Wasser >20 Gew.-% |

**Tab. 3: Gewichtsverhältnisse der Komponenten (A) und (D)**

| Beispiel | Verhältnis (A) : (D) |
|---|---|
| Beispiel 1 | 5:1 |
| Beispiel 2 | 4,6:1 |
| Beispiel 3 | 5,4:1 |
| Beispiel 4 | 4,6:1 |
| Vergleichsbeispiel 1 | 4,2:1 |
| Vergleichsbeispiel 2 | 5,4:1 |
| Vergleichsbeispiel 3 | keine Komponente (D), kein Glycerid |
| Vergleichsbeispiel 4 | 5,4:1 |

**Tab. 4: Gewichtsverhältnisse der Komponenten (E) und (F)**

| Beispiel | Verhältnis (E) : (F) |
|---|---|
| Beispiel 1 | 1:1,3 |
| Beispiel 2 | 1:2,2 |
| Beispiel 3 | 1:3 |
| Beispiel 4 | 1:2,2 |
| Vergleichsbeispiel 1 | 1:4,9 |
| Vergleichsbeispiel 2 | 1: 8 |
| Vergleichsbeispiel 3 | 1:1,1 |
| Vergleichsbeispiel 4 | 1:1 |

**Tab 5: Emulsionskonzentrat -Verdünnungen. Alle Angaben in Gew.% Aktivsubstanz bezogen auf das Gesamtgewicht der Zubereitung.**

| **Komponente** | **1** | **2** | **3** | **4** | **5** | **6** | **7** | **8** | **9** |
|---|---|---|---|---|---|---|---|---|---|
| | | | | | | | | | |
| Emulsionskonzentrat Bsp. 1 * | 5.0 | 4.0 | 7.5 | 3.5 | 6.5 | 2.0 | 7.5 | 9.0 | 5.5 |
| Euxyl^{®} K 300 (Phenoxyethanol, Methyl-paraben, Ethylparaben, Propylparaben, Butylparaben, isobutylparaben) | | | | 0.8 | | | 0.8 | | |
| Nipaguard^{®} BPX (Phenoxy-ethanol, Methyl-paraben, Propyl-paraben, 2-Bromo-2-Nitro-propane-1,3-Diol) | | | 0.5 | | | | | | |
| Sodium Benzoate | 0.5 | | | | | | | | 0.5 |
| Potassium Sorbate | | | | | | | | | 0.2 |
| Euxyl PE^{®} 9010 (Phenoxyethanol, Ethylhexylglycerin) | | | | | | 1.0 | | | |
| Nipaguard^{®} PO 5 (Phenoxyethanol, Piroctone Olamine) | | 1.0 | | | 1.0 | | | 1.0 | |
| Sensiva SC 10 (Caprytyl Glycol, Ethythexylglycerin) | 0.2 | | | | | 0.1 | | | |
| Herbalia^{®} Green Tea (Camellia sinensis and Silica) | | | | | | 0.05 | | | |
| Cosmedia^{®} SP (Sodium Polyacrylate) | | 0.3 | | 0.2 | | | | | |
| Melhydran^{®} LS 4420 (Honey extract) | | | 0.5 | | | | | | |
| Lactolan^{®} LS 5879 (Hydrolyzed Milk Protein) | | | 0.5 | | | | | | |
| Copherol^{®} 1250 C (Tocopheryl Acetate) | | | | | 0.4 | | | | |
| Dihydroxyacetone (DHA) | | | | | | | | | 2.0 |
| Insect Repellent 3535^{®} (Ethyl Butylacetylaminopropionate) | | | | | | | | 10.0 | |
| Eusolex^{®} OCR (Octocrylene) | | | | | | | 1.0 | | |
| Eusolex^{®} 9020 (Butyl Methoxydibenzoylmethane) | | | | | | | 0.3 | | |
| Myritol^{®} 318 (Caprylic/Capric riglyceride) | | | | 0.1 | | | | | |
| Perfume | | 0.1 | 0.2 | | 0.3 | | 0.1 | | 0.25 |
| Wasser | add 100 | add 100 | add 100 | add 100 | add 100 | add 100 | add 100 | add 100 | add 100 |
| pH-Wert der Verdünnung (ggf. | 4.2 - | 6.0 - | 4.2 - | 6.0 - | 6.0 - | 6.0 - | 6.0 - | 6.0-6.5 | 3.5 - |
| mit pH-Adjuster einstellen) | 5.0 | 7.0 | 5.0 | 6.5 | 6.5 | 6.5 | 6.5 | | 4.0 |

| | | | | | | | | | |
|---|---|---|---|---|---|---|---|---|---|
| * Alternativ können anstelle des Emulsionskonzentrats nach Bsp. 1 auch die von Bsp. 2, Bsp.3 oder Bsp. 4 verwendet werden. | | | | | | | | | |

## Patentansprüche

1. Emulsionskonzentrat, enthaltend folgende Komponenten:
(A) Alkyl- und/oder Alkenyloligoglykosid(e) mit 8 bis 22 Kohlenstoffatomen im Alk(en)ylrest,
(B) Polyglycerolester,
(C) Polyol(e),
(D) Glycerid(e),
(E) wasserunlösliche Komponente(n) mit einer Polarität von etwa 30-40 mN/m und
(F) wasserunlösliche Komponente(n) mit einer Polarität von etwa 15-25 mN/m,
wobei
die Komponenten (A), (B) und (C) ein Gewichtsverhältnis A: B : C von 1 : (0,9-1,1): (0,9-1,1) aufweisen,
die Komponenten (A) und (D) ein Gewichtsverhältnis A : D von 6:1 1 bis 3:1 1 aufweisen,
die Komponenten (E) und (F) ein Gewichtsverhältnis E : F von 1:1 1 bis 1 : 3 aufweisen, und der Wasseranteil des Emulsionskonzentrats 10 bis 20 Gew.-% beträgt.

2. Emulsionskonzentrat nach Anspruch 1,
weiches als Komponente (A) ein Alkyloligoglykosidgemisch mit einem Verhältnis von C12-Alkylrest zu C14-Alkylrest von etwa 7 : etwa 3 aufweist.

3. Emulsionskonzentrat nach Anspruch 1 oder 2,
welches als Komponente (B) einen Polyglycerolester einer Polyhydroxystearinsäure aufweist.

4. Emulsionskonzentrat nach einem der vorhergehenden Ansprüche,
welches als Komponente (C) ein Polyol mit 2 bis 6 Hydroxylgruppen aufweist.

5. Emulsionskonzentrat nach Anspruch 4, **dadurch gekennzeichnet, dass** die Polyole 2 bis 18 C-Atome enthalten.

6. Emulsionskonzentrat nach einem der vorhergehenden Ansprüche,
welches als Komponente (D) ein Glycerinoleat mit mehr als 80 Gew.-% ungesättigten C18-Fettsäuren aufweist, wobei der Monoesteranteil mehr als 90 Gew.-% beträgt.

7. Emulsionskonzentrat nach einem der vorhergehenden Ansprüche, **dadurch gekennzeichnet, dass** es keine ethoxylierten Substanzen enthält.

8. Emulsionskonzentrat nach einem der vorhergehenden Ansprüche, **dadurch gekennzeichnet, dass** es eine Viskosität bei 15°C von unterhalb 30000 mPa*s aufweist

9. Verwendung des Emulsionskonzentrats nach einem der Ansprüche 1 bis 8 als kosmetische oder pharmazeutische Zubereitung.

10. Verwendung des Emulsionskonzentrats nach einem der Ansprüche 1 bis 8 zur Herstellung kosmetischer oder pharmazeutischer Zubereitungen, insbesondere Öl-in-Wasser-Emulsionen.

11. Kosmetische oder pharmazeutische Zubereitung, erhältlich durch Verdünnen des Emulsionskonzentrats nach einem der Ansprüche 1 bis 8.

12. Verwendung der Emulsionskonzentrate nach einem der Ansprüche 1 bis 8 oder der kosmetischen oder pharmazeutischen Zubereitungen nach Anspruch 11 zur Beschichtung von Substraten.

13. Papier-, Vlies- oder Gewebe-Produkt zur Körperpflege und/oder -reinigung, welches das Emulsionskonzentrat nach einem der Ansprüche 1 bis 8 oder die Zubereitung nach Anspruch 11 aufweist.
